# EUROPEAN PATENT SPECIFICATION

(11) **EP 2 857 057 B1**
(45) Date of publication and mention of the grant of the patent: **27.05.2020**
(21) Application number: 12877800.8
(22) Date of filing: 30.05.2012
(51) Int. Cl.: A61M 25/00

(54) **MEDICAL TUBE AND METHOD FOR PRODUCING SAME**
MEDIZINISCHE RÖHRE UND VERFAHREN ZUR HERSTELLUNG DAVON
TUBE MÉDICAL ET PROCÉDÉ DE PRODUCTION ASSOCIÉ

(43) Date of publication of application: 08.04.2015
(73) Proprietor: Terumo Kabushiki Kaisha, Tokyo 151-0072 (JP)
(72) Inventor: TANIOKA, Hiromichi, Fujinomiya-shi Shizuoka 418-0015 (JP)
(74) Representative: Casalonga
(86) International application number: PCT/JP2012/063916
(87) International publication number: WO 2013/179411

(56) References cited:
- EP-A1- 0 365 993
- EP-A1- 1 935 614
- JP-A- H05 184 664
- JP-A- H10 156 942
- JP-A- 2001 120 655
- US-A- 4 795 434
- US-A1- 2011 224 650

## Description

### Technical Field

The present invention relates to a medical tube and a method for producing the same.

### Background Art

In the related art, an ultrasonic catheter having an imaging function is inserted into a blood vessel such as a coronary artery of the heart, or a vessel such as a bile duct, thereby allowing imaging diagnosis to be performed.

An imaging apparatus for diagnosis includes an intravascular ultrasound (IVUS) diagnostic apparatus. The intravascular ultrasound diagnostic apparatus generally has a configuration in which a probe incorporating an ultrasonic transducer is caused to perform radial scanning in a blood vessel, the same ultrasonic transducer receives a reflected wave (ultrasonic echo) reflected on a biological tissue, and then, a cross-sectional image of the blood vessel is visualized, based on intensity of the ultrasonic echo generated through processes such as amplification and demodulation.

In addition, as the imaging apparatus for diagnosis, an optical coherence tomography (OCT) diagnostic apparatus is also used. The optical coherence tomography diagnostic apparatus is configured so that a catheter incorporating an optical fiber having an attached probe incorporating an optical lens and an optical mirror at a distal end is inserted into a blood vessel, the optical mirror arranged on the distal side of the optical fiber is caused to perform radial scanning, light is emitted into the blood vessel, and the cross-sectional image of the blood vessel is visualized based on reflected light from the biological tissue. Furthermore, recently, an imaging apparatus for diagnosis which employs optical frequency domain imaging (OFDI) called next generation OCT has been proposed.

As an example of the imaging apparatus for diagnosis, for example, PTL 1 discloses an intravascular ultrasound diagnostic apparatus which employs an ultrasonic catheter in which a nested structure flexible in an axial direction is provided on a proximal side of a sheath. An ultrasonic transducer inside the sheath can be moved in the axial direction with respect to the sheath by changing the total length of a catheter main body. Then, in a state where the ultrasonic transducer is arranged on the distal side inside the sheath, only the ultrasonic transducer is pulled back so as to pass through a target lesion while the sheath is left behind. In this manner, it is possible for an ultrasonic image continuously acquired all around the target lesion to be observed, or to create three-dimensional data relating to a shape of a blood vessel and a duct.

Document EP0365993 discloses a further example of a medical tube for a catheter and a method of manufacturing the same. This document does not disclose the features of the characterising portion of claims 1 and 5.

### Citation List

### Patent Literature

PTL 1: Pamphlet of International Publication No. 2007/001956

### Summary of Invention

### Technical Problem

Incidentally, in many cases, the medical tube as described above has a configuration including a tube body molded by means of extrusion molding which enables an elongated structure to be easily molded. In some cases, for example, this tube body is fitted and connected to a hole portion of a connector made of resin which is an injection-molded component. However, an extrusion-molded component is inferior to an injection-molded component in dimensional accuracy. Therefore, in view of respective dimensional tolerances, a design value of a clearance in a fitting portion inevitably increases so that even an extrusion-molded component having the largest outer diameter within a tolerance range can be fitted to the hole portion of the injection-molded component having the smallest inner diameter within the tolerance range. If the clearance in the fitting portion of the two components increases, in the fitting portion, axes of the two components deviate from each other, relative positions of the two components become tilted, or lengths for fitting are changed. If the relative positions deviate in this way, for example, in the medical tube including a drive shaft of the above-described ultrasonic catheter, there is a possibility that sliding resistance occurring locally between the drive shaft and other members may result in uneven rotation, when the drive shaft is rotated at a high speed. If uneven rotation occurs, when an image is displayed during diagnosis with a catheter, characteristics which are anatomically different from those of the original lumen are displayed undesirably. As described above, the deviation of the relative positions of configuration components may cause degraded performance of the medical tube, may cause difficulties in automating an assembly process, and may cause degraded productivity.

The present invention is made in order to solve the above-described problem, and an object thereof is to provide a medical tube and a method for producing the same which can prevent performance and productivity from being degraded even when an extrusion-molded component and an injection-molded component are fitted to each other.

### Solution to Problem

A medical tube according to claim 1 and a method for producing a medical tube according to claim 5 achieve the above-described object

### Advantageous Effects of Invention

In the medical tube configured as described above, the outer peripheral surface of the end portion of the extrusion-molded tube body is molded by means of thermal processing in a mold after extrusion molding. Accordingly, the outer peripheral surface of the tube body has improved dimensional accuracy, and can be fitted to the containing body using a more desirable clearance. Therefore, it is possible to prevent performance and productivity of the medical tube from being degraded.

In the method for producing the medical tube configured as described above, the tube body produced in such a way that the outer peripheral surface of the end portion of the extrusion-molded tubular workpiece is molded into a different shape by means of thermal processing is fitted to the containing body. Accordingly, the outer peripheral surface of the tube body has improved dimensional accuracy, and can be fitted to the containing body using a more desirable clearance. Therefore, it is possible to prevent performance and productivity of the medical tube from being degraded.

If the outer peripheral surface of the end portion of the tube body is adapted to have at least one groove portion extending along the axial direction of the tube body, when the tube body is fitted to the containing body, protruding portions formed between the groove portions can be respectively deformed, thereby easily decreasing positional deviation by causing axes of the tube body and the containing body to coincide with each other. In addition, when the tube body is fitted to a containing portion, the groove portion decreases frictional resistance, thereby facilitating the fitting. In addition, since the groove portion is provided, it is easy to spread an adhesive throughout a fitting portion. Moreover, excess adhesive can be released in the groove portion, thereby enabling favorable adhesion.

If the outer peripheral surface of the end portion of the tube body is adapted to have a rotational symmetric shape in a cross section orthogonal to the axial direction of the tube body, when the tube body is fitted to the containing body, protruding portions formed between the groove portions can be respectively uniformly deformed, thereby easily decreasing positional deviation by causing axes of the tube body and the containing body to coincide with each other.

If there is further provided a drive shaft that rotatably penetrates the inside of the tube body and the containing body, to an end portion of which a signal transmitting and receiving portion for acquiring image information is fixed, and that transfers a mechanical drive force to the signal transmitting and receiving portion, positional deviation decreases between the tube body and the containing body. Accordingly, when the drive shaft is rotated, sliding resistance is less likely to occur locally between the drive shaft and other members, thereby preventing uneven rotation. It is possible to display characteristics which are anatomically close to those of the original lumen.

If the medical tube is adapted to further have a sheath in which the signal transmitting and receiving portion and the drive shaft are arranged to be movable thereinside, and which is inserted into a lumen, a hub which moves while holding the drive shaft so as to move the drive shaft in the axial direction of the sheath, a first tube body which is arranged on a proximal side of the sheath,
and a second tube body which is arranged on a distal side of the hub, and which can relatively move an inner portion or an outer portion of the first tube body with respect to the first tube body in accordance with a movement of the hub, even when the first tube body and the second tube body are relatively moved, it is possible to maintain proper axes and positions. Accordingly, the medical tube for imaging diagnosis is provided with improved performance.

If in the process of producing the tube body, at least one groove portion extending along the axial direction of the tube body is formed on the outer peripheral surface of the end portion of the tube body, when the tube body is fitted to the containing body, protruding portions formed between the groove portions can be respectively deformed, thereby easily decreasing positional deviation by causing axes of the tube body and the containing body to coincide with each other. In addition, when the tube body is fitted to the containing portion, the groove portion decreases frictional resistance, thereby facilitating the fitting. In addition, since the groove portion is provided, it is easy to spread the adhesive throughout the fitting portion, when the adhesive is used. Moreover, excess adhesive can be released in the groove portion, thereby enabling favorable adhesion.

If in the process of producing the tube body, the outer peripheral surface of the end portion of the tube body is formed into a rotational symmetric shape in a cross section orthogonal to the axial direction of the tube body, when the tube body is fitted to the containing body, protruding portions formed between the groove portions can be respectively uniformly deformed, thereby easily decreasing positional deviation by causing axes of the tube body and the containing body to coincide with each other.

If the method for producing the medical tube is adapted to further include a process of arranging a drive shaft that rotatably penetrates the inside of the tube body and the containing body, to an end portion of which a signal transmitting and receiving portion for acquiring image information is fixed, and that transfers a mechanical drive force to the signal transmitting and receiving portion, so as to rotatably penetrate the inside of the tube body and the containing body, positional deviation decreases between the tube body and the containing body. Accordingly, when the drive shaft is rotated, sliding resistance is less likely to occur locally between the drive shaft and other members, thereby preventing uneven rotation. It is possible to display characteristics which are anatomically close to those of the original lumen.

If there is provided the method for producing a medical tube including a sheath in which the signal transmitting and receiving portion and the drive shaft are arranged to be movable thereinside, and which is inserted into a lumen, a hub which moves while holding the drive shaft so as to move the drive shaft in the axial direction of the sheath, a first tube body which is arranged on a proximal side of the sheath, and a second tube body which is arranged on a distal side of the hub, and which can relatively move an inner portion or an outer portion of the first tube body with respect to the first tube body in accordance with a movement of the hub, even when the first tube body and the second tube body are relatively moved, it is possible to maintain proper axes and positions. Accordingly, the medical tube for imaging diagnosis is provided with improved performance.

### Brief Description of Drawings

[Fig. 1] Fig. 1 is a plan view illustrating a medical tube according to an embodiment of the present invention.
[Fig. 2] Fig. 2 is a plan view illustrating a luminal diagnosis system.
[Fig. 3] Fig. 3 is a longitudinal sectional view illustrating a distal portion of a medical tube.
[Fig. 4] Fig. 4 is a plan view illustrating a medical tube when a transducer unit is pulled back.
[Fig. 5] Fig. 5 is a longitudinal sectional view illustrating a hub of a medical tube.
[Fig. 6] Fig. 6 is a cross-sectional view taken along line A-A in Fig. 5.
[Fig. 7] Fig. 7 is a longitudinal sectional view illustrating a unit connector and a relay connector of a medical tube.
[Fig. 8] Fig. 8 is a cross-sectional view taken along line B-B in Fig. 7.
[Fig. 9] Fig. 9 is a cross-sectional view taken along line C-C in Fig. 7.
[Fig. 10] Fig. 10 is a side view illustrating a processing apparatus for processing a workpiece.
[Fig. 11] Fig. 11 is a longitudinal sectional view illustrating a mold of a processing apparatus.
[Fig. 12] Fig. 12 is a cross-sectional view taken along line D-D in Fig. 11.
[Fig. 13] Fig. 13 is a side view of a processing apparatus illustrated when a workpiece is pressed into a mold in a state where a first movement portion and a second movement portion are connected to each other.
[Fig. 14] Fig. 14 is a side view illustrated when the workpiece is pressed into the mold after the first movement portion and the second movement portion are separated from each other.
[Fig. 15] Fig. 15 is a longitudinal sectional view illustrated when the workpiece is thermally processed inside the mold.
[Fig. 16] Fig. 16 is a side view of the processing apparatus illustrated when thermal processing is completed.
[Fig. 17] Fig. 17 is a side view of the processing apparatus illustrated when the first movement portion and the second movement portion return to the original position.
[Fig. 18] Fig. 18 is a perspective view illustrating a distal portion of an outer tube molded by means of the thermal processing in the mold.

### Description of Embodiments

Hereinafter, an embodiment of the present invention will be described with reference to the drawings. In some cases, dimensional proportions in the drawings may be exaggerated and different from actual proportions for convenience of description.

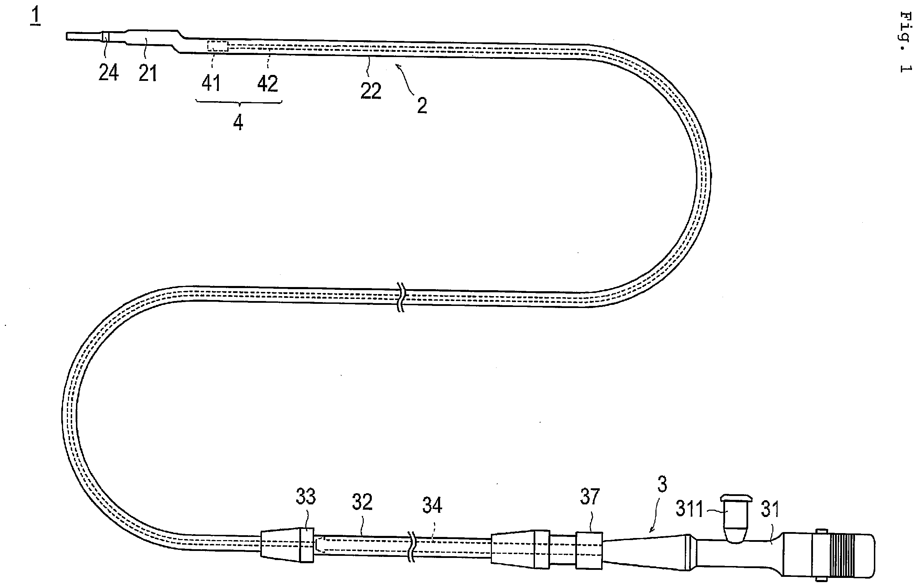

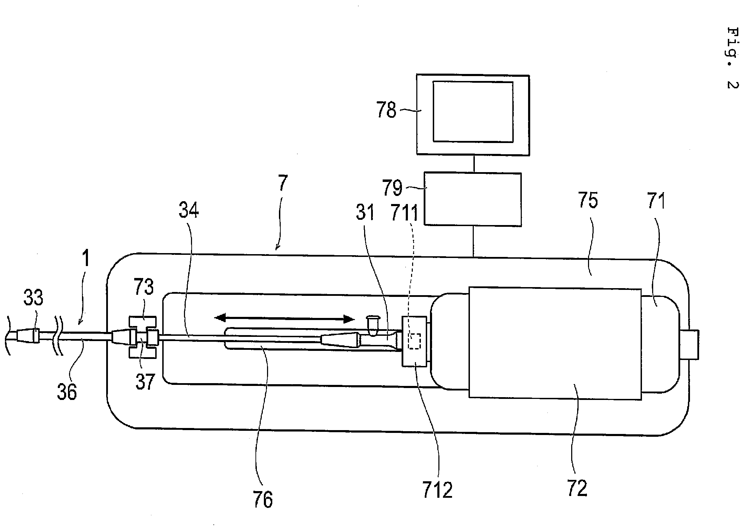

As illustrated in Figs. 1 and 2, a medical tube 1 according to the present embodiment is an ultrasonic catheter which internally accommodates an imaging core 4 for ultrasonic diagnosis and is inserted into a lumen. The medical tube 1 is connected to an external drive device 7 which holds the medical tube 1 and drives the imaging core 4, and is mainly used for diagnosing internal conditions of the blood vessel. In this description, a side inserted into a lumen of a living body is referred to as a "distal end" or a "distal side", and an operating hand side is referred to as a "proximal end" or a "proximal side".

The medical tube 1 includes a sheath 2 inserted into the lumen, the imaging core 4 which transmits and receives an ultrasonic wave toward and from a tissue inside the lumen, and an operation unit 3 through which the imaging core 4 penetrates and which is located on the further proximal side from the sheath 2.

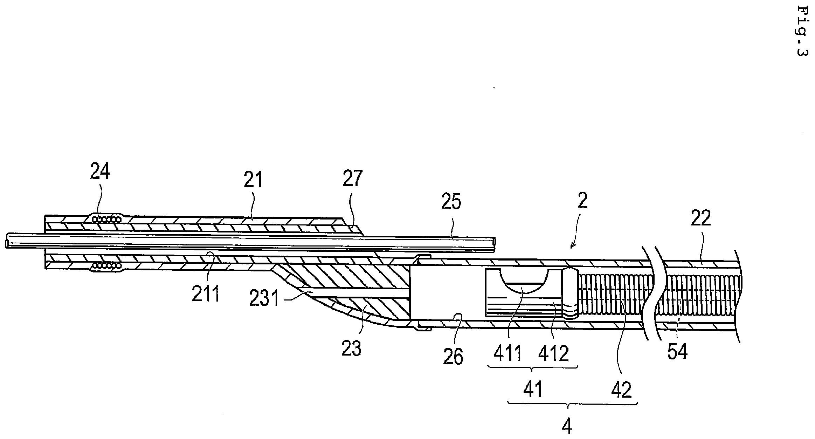

As illustrated in Fig. 3, the sheath 2 has a sheath distal portion 21, a sheath tube 22, and a filling liquid inlet and outlet member 23.

A cylindrical sheath distal member 27 in which a guide wire lumen 211 is formed, and an X-ray contrast marker 24 disposed in a portion which is located on the proximal side slightly away from the distal portion are formed in the sheath distal portion 21. The guide wire 25 is inserted into the lumen in advance, and the medical tube 1 is guided to a target lesion while the guide wire 25 passes through the guide wire lumen 211. The X-ray contrast marker 24 is disposed so that a distal position of the medical tube 1 can be identified under X-ray illumination when the medical tube 1 is inserted into the lumen.

A priming port 231 serving as a hole which communicates with a lumen 26 inside the sheath tube 22 and causes physiological saline internally filling the sheath tube 22 to flow outward is formed in the filling liquid inlet and outlet member 23.

The imaging core 4 is incorporated inside the sheath 2 so as to be slidable in the axial direction of the sheath 2. The imaging core 4 includes a transducer unit 41 which transmits and receives the ultrasonic wave toward and from the tissues inside the lumen, and a drive shaft 42 which rotates the transducer unit 41 and which is attached to a distal end thereof. The transducer unit 41 is configured to have an ultrasonic transducer 411 (signal transmitting and receiving portion) which transmits and receives the ultrasonic wave, and a housing 412 which accommodates the ultrasonic transducer 411.

The sheath tube 22 is formed of a material having high transmittance properties for the ultrasonic wave. A portion inside a moving range of the ultrasonic transducer 411 of the sheath 2 configures an acoustic window portion through which the ultrasonic wave is transmitted.

The drive shaft 42 is flexible, and moreover, has properties of being able to transfer rotational power generated in the operation unit 3 (refer to Fig. 1) to the transducer unit 41. For example, the drive shaft 42 is configured to have a tubular body in a shape of a multi-layer coil such as a three-layer coil in which the layers are alternately formed in rightward, leftward, and rightward winding directions. The drive shaft 42 transfers the rotational power so as to rotate the transducer unit 41. In this manner, it is possible to observe the target lesion inside the lumen such as a blood vessel and a duct at a viewing angle of 360 degrees. In addition, the drive shaft 42 allows a signal line 54 to pass therethrough in order to transmit a signal detected by the transducer unit 41 to the operation unit 3.

As illustrated in Fig. 1, the operation unit 3 has a hub 31 having a port 311 through which the physiological saline for air venting is injected, a unit connector 37 which is connected to the hub 31 via an inner tube 34 (second tube body), and a relay connector 33 which is connected to the unit connector 37 via an outer tube 32 (first tube body) and connects the sheath 2 and the operation unit 3 to each other.

The hub 31 holds the drive shaft 42 and the inner tube 34. The inner tube 34 is pressed into the unit connector 37 and the outer tube 32, or is drawn out therefrom. In conjunction therewith, the drive shaft 42 slides inside the sheath 2 in the axial direction.

When the inner tube 34 is pressed furthest into the outer tube 32, the sheath side end portion of the inner tube 34 reaches the vicinity of the sheath side end portion of the outer tube 32, that is, the vicinity of the relay connector 33, as illustrated in Fig. 1. Then, in this state, the transducer unit 41 is located in the vicinity of the distal end of the sheath tube 22 of the sheath 2.

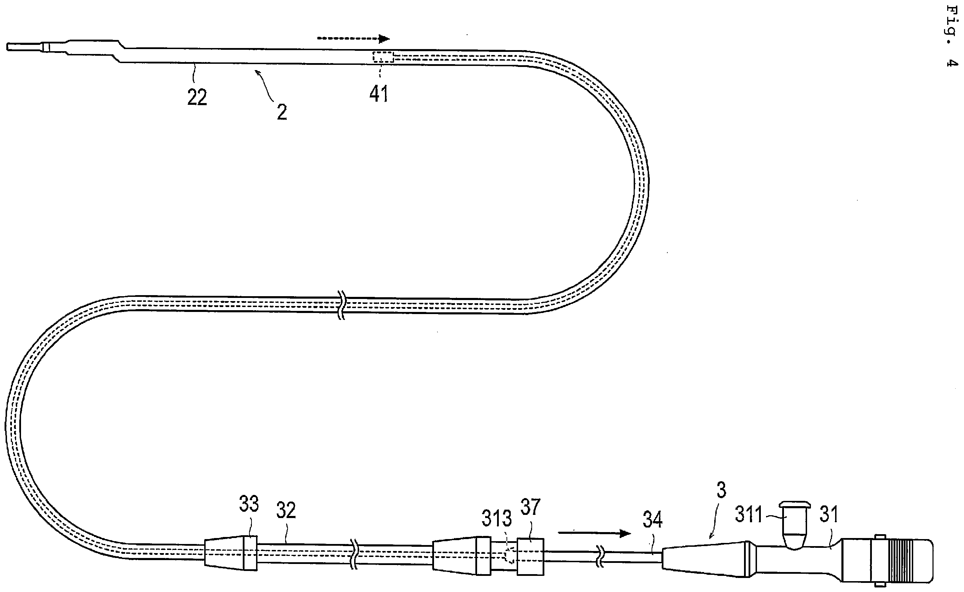

In addition, when the inner tube 34 is drawn out from the outer tube 32, a stopper 313 formed at the distal end of the inner tube 34 catches on an inner wall of the unit connector 37, and a portion other than the vicinity of the caught distal end is exposed, as illustrated in Fig. 4. Then, in this state, the transducer unit 41 is pulled back thereinside while the sheath 2 is left behind. The transducer unit 41 moves while being rotated. In this manner, it is possible to create a tomographic image of the blood vessel and the duct.

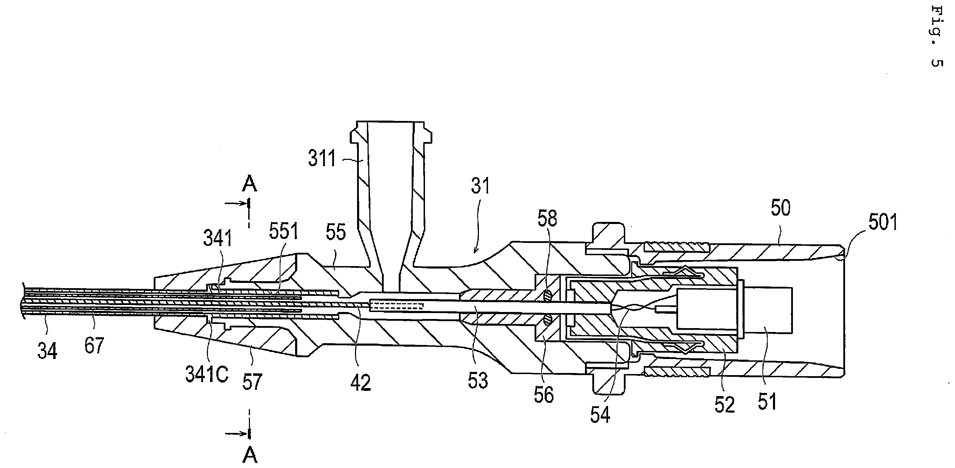

As illustrated in Fig. 5, the hub 31 of the operation unit 3 has a joint 50, a male connector 51, a rotor 52, a connection pipe 53, a signal line 54, a hub main body 55, a seal member 56, and a kink resistance protector 57.

The joint 50 has an opening portion 501 on a user's hand side of the medical tube 1, and the male connector 51 and the rotor 52 are arranged thereinside. The male connector 51 can be connected to a female connector 711 (refer to Fig. 1) included in the external drive device 7, through the opening portion 501 side of the joint 50. In this manner, the external drive device 7 and the male connector 51 are mechanically and electrically connected to each other.

The rotor 52 holds the connection pipe 53 so as not to be rotatable, and rotates integrally with the male connector 51. The connection pipe 53 holds the drive shaft 42 at the end portion opposite to the rotor 52 side in order to transfer the rotation of the rotor 52 to the drive shaft 42. The signal line 54 passes through the inside of the connection pipe 53. The signal line 54 is configured so that one end is connected to the male connector 51 and the other end is connected to the transducer unit 41 after passing through the inside of the drive shaft 42. Observation results in the transducer unit 41 are transmitted to the external drive device 7 via the male connector 51, and are displayed as an image after being subjected to proper processing.

The hub main body 55 guides the physiological saline injected through the port 311 into the inner tube 34 without the physiological saline leaking out. The seal member 56 including an O-ring 58 is installed between the hub main body 55 and the joint 50. Accordingly, there is no possibility that the physiological saline may leak out to the opening portion 501 side of the joint 50.

The proximal portion of the inner tube 34 is inserted into the hub main body 55 (containing body) so that an outer peripheral surface of the inner tube 34 is fitted to an inner peripheral surface 551 thereinside. The kink resistance protector 57 is arranged around the inner tube 34 and the hub main body 55.

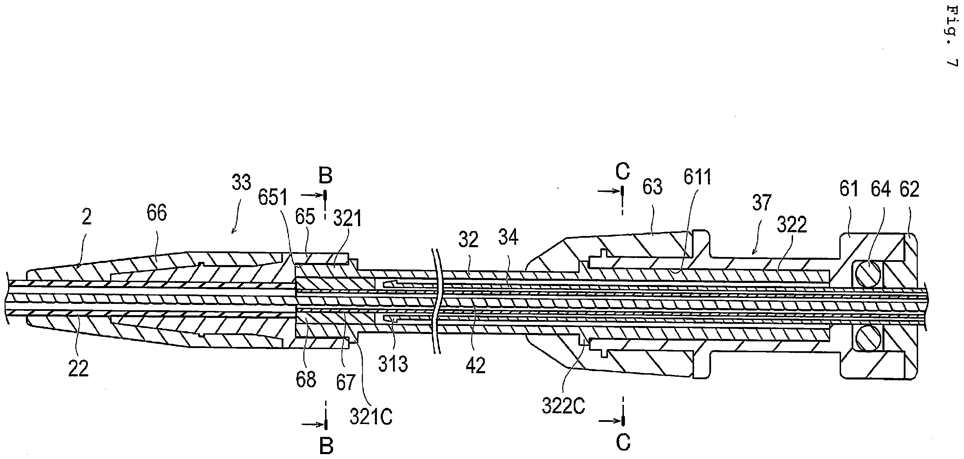

As illustrated in Fig. 7, the unit connector 37 has a unit connector main body 61, a sealing member 62, a cover member 63, and a packing 64.

The unit connector main body 61 (containing body) is inserted so that the proximal portion of the outer tube 32 attached to the relay connector 33 is fitted to an inner peripheral surface 611 thereinside. The inner tube 34 extending from the hub 31 is inserted into the outer tube 32. The sealing member 62 holds the packing 64 in combination with the unit connector main body 61, and the cover member 63 holds the outer tube 32 in combination with the unit connector main body 61. The packing 64 seals a portion between the unit connector main body 61 and the sealing member 62. Accordingly, even when the physiological saline supplied to the port 311 of the hub 31 passes through the inner tube 34 and flows into the outer tube 32, there is no possibility that the physiological saline may leak out from the unit connector 37.

In addition, the inner tube 34 extending from the hub 31 has the stopper 313 formed at the distal end thereof. Accordingly, even when the hub 31 is pulled furthest out therefrom, that is, even when the inner tube 34 is pulled furthest out from the outer tube 32, there is no possibility that the stopper 313 may catch on the inner wall of the unit connector main body 61 and the inner tube 34 is removed from the unit connector 37.

The relay connector 33 has an outer tube holding portion 65 (containing body) and a kink resistance protector 66. A portion of the outer tube 32 is inserted into the outer tube holding portion 65 so that an outer peripheral surface of the distal portion of the outer tube 32 is fitted to an inner peripheral surface 651 thereinside. In addition, a proximal side end portion of the sheath tube 22 is connected to the inner peripheral surface 651 of the outer tube holding portion 65, where a path for guiding the drive shaft 42 and the physiological saline which pass through the outer tube 32 to the sheath 2 is formed. The sheath tube 22 is adapted to have a single-layer structure in Fig. 7, but may be a multi-layer structure.

A spacer tube 68 through which the drive shaft 42 passes is arranged inside the distal portion of the outer tube 32 fitted to the outer tube holding portion 65. A protection tube 67 is fixed to an inner wall of the spacer tube 68. The protection tube 67 extends toward the inside of the inner tube 34 extending from the hub 31, and is arranged between the drive shaft 42 and the inner tube 34. Therefore, when the inner tube 34 is pressed into the outer tube 32, the protection tube 67 is pressed into the inner tube 34 in a direction opposite to the pressing direction. When the inner tube 34 is pressed into or pulled out from the outer tube 32, the protection tube 67 is also relatively pressed into or pulled out from the inner tube 34 in the opposite direction. Accordingly, even when the outer tube 32 comes into contact with the inner tube 34 causing friction therebetween and generating a bending force in the drive shaft 42, the protection tube 67 suppresses the bending force, and can prevent the drive shaft 42 from being bent. The protection tube 67 is formed of a tubular body having a coarsely wound metal coil shape. Therefore, the physiological saline can flow into the protection tube 67 through a gap in the coil. Accordingly, there is no possibility that air may remain inside the outer tube 32.

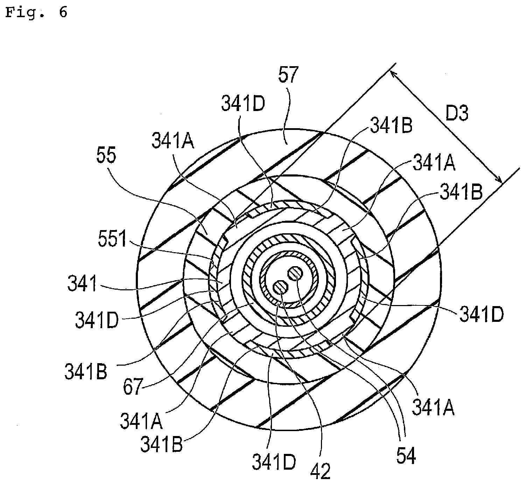

The outer tube 32 and the inner tube 34 which are described above are formed into a cylindrical shape by means of a known extrusion molding method. Then, as illustrated in Figs. 7 to 9, in the outer tube 32, an outer peripheral surface of an outer tube distal portion 321 which is fitted to the inner peripheral surface 651 of the outer tube holding portion 65 (containing body) and an outer peripheral surface of an outer tube proximal portion 322 which is fitted to the inner peripheral surface 611 of the unit connector main body 61 (containing body) are formed into a different shape by being heated inside a separately provided mold (to be described later) after extrusion molding. In addition, as illustrated in Figs. 5 and 6, in the inner tube 34, an outer peripheral surface of an inner tube proximal portion 341 which is fitted to the inner peripheral surface 551 of the hub main body 55 (containing body) is formed into a different shape by being heated inside a separately provided mold (to be described later) after extrusion molding.

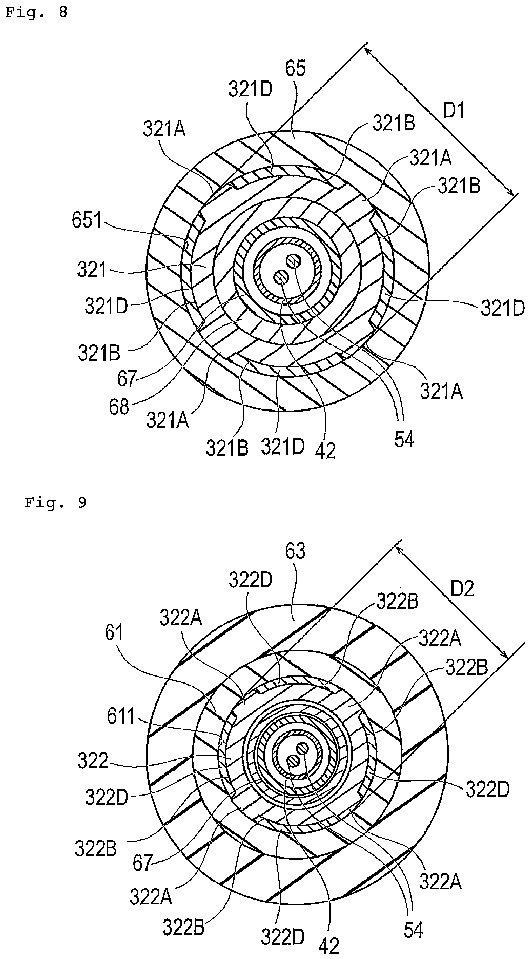

As illustrated in Fig. 8, in the outer tube distal portion 321, four projection portions 321A configuring an outer peripheral surface fitted to the inner peripheral surface 651 of the outer tube holding portion 65 are arrayed side by side at equal intervals in a circumferential direction. Four groove portions 321B are formed between the four projection portions 321A. The outer tube distal portion 321 has a rotational symmetric shape in a cross section orthogonal to an axis of the outer tube 32. An annular portion 321C produced during thermal processing using a mold is formed on a proximal side of the projection portions 321A and the groove portions 321B. The annular portion 321C may not be formed, or may be removed if necessary. An adhesive 321D for adhering to the outer tube holding portion 65 is interposed between the groove portions 321B. An outer diameter D1 configured to include the four projection portions 321A has further improved dimensional accuracy after the extrusion molding, since the four projection portions 321A are molded by means of thermal processing using a mold. Therefore, it is possible to minimize a clearance between the outer tube distal portion 321 and the inner peripheral surface 651 of the outer tube holding portion 65 which is an injection-molded component. As an example, the outer diameter D1 of the outer tube distal portion 321 is 2.0 mm to 4.0 mm, and the clearance between the outer tube distal portion 321 and the inner peripheral surface 651 of the outer tube holding portion 65 can be set to 0.1 mm to 0.5 mm.

As illustrated in Fig. 9, in the outer tube proximal portion 322, four projection portions 322A configuring an outer peripheral surface fitted to the inner peripheral surface 611 of the unit connector main body 61 are arrayed side by side at equal intervals in the circumferential direction. Four groove portions 322B are formed between the four projection portions 322A. The outer tube proximal portion 322 has a rotational symmetric shape in a cross section orthogonal to the axis of the outer tube 32. An annular portion 322C produced during thermal processing using a mold is formed on a distal side of the projection portions 322A and the groove portions 322B. The annular portion 322C may not be formed, or may be removed if necessary. An adhesive 322D for adhering to the unit connector main body 61 is interposed between the groove portions 322B. An outer diameter D2 configured to include the four projection portions 322A has further improved dimensional accuracy after the extrusion molding, since the four projection portions 322A are molded by means of thermal processing using a mold. Therefore, it is possible to minimize a clearance between the outer tube proximal portion 322 and the inner peripheral surface 611 of the unit connector main body 61 which is an injection-molded component. As an example, the outer diameter D2 of the outer tube proximal portion 322 is 2.0 mm to 4.0 mm, and the clearance between the outer tube proximal portion 322 and the inner peripheral surface 611 of the unit connector main body 61 can be set to 0.1 mm to 0.5 mm.

As illustrated in Fig. 6, in the inner tube proximal portion 341, four projection portions 341A configuring an outer peripheral surface fitted to the inner peripheral surface 551 of the hub main body 55 are arrayed side by side at equal intervals in the circumferential direction. Four groove portions 341B are formed between the four projection portions 341A. The inner tube proximal portion 341 has a rotational symmetric shape in a cross section orthogonal to an axis of the inner tube 34. An annular portion 341C produced during thermal processing using a mold is formed on a distal side of the projection portions 341A and the groove portions 341B. The annular portion 341C may not be formed, or may be removed if necessary. An adhesive 341D for adhering to the hub main body 55 is interposed between the groove portions 341B. An outer diameter D3 configured to include the four projection portions 341A has further improved dimensional accuracy after the extrusion molding, since the four projection portions 341A are molded by means of thermal processing using a mold. Therefore, it is possible to minimize a clearance between the inner tube proximal portion 341 and the inner peripheral surface 551 of the hub main body 55 which is an injection-molded component. As an example, the outer diameter D3 of the inner tube proximal portion 341 is 1.0 mm to 3.0 mm, and the clearance between the inner tube proximal portion 341 and the inner peripheral surface 551 of the hub main body 55 can be set to 0.1 mm to 0.5 mm.

The outer tube 32 and the inner tube 34 are made of polyethylene. However, the material is not particularly limited thereto as long as the material is a thermoplastic resin which can be extrusion-molded. In addition, the spacer tube 68 is also made of polyethylene, but the material is not particularly limited thereto.

As illustrated in Fig. 2, the above-described medical tube 1 is connected to and driven by the external drive device 7. On a base 75, the external drive device 7 includes a drive unit 71 which has an incorporated external drive source such as a motor and rotatably drives the drive shaft 42, moving means 72 for gripping and moving the drive unit 71 in the axial direction by using the motor, and a holding portion 73 which holds a portion of the medical tube 1 so as to be fixedly positioned. The external drive device 7 is connected to a control unit 79 which controls the drive unit 71 and the moving means 72. An image obtained by the transducer unit 41 is displayed on a display unit 78 connected to the control unit 79.

The moving means 72 can grip and fix the drive unit 71, and is a feeding mechanism which can move the gripped drive unit 71 forward and backward along a groove rail 76 on the base 75.

The drive unit 71 has the driving female connector 711 to which the driving male connector 51 of the medical tube 1 can be connected, and a joint connection portion 712 which can be connected to the joint 50 of the medical tube 1. The connection enables a signal to be transmitted and received to and from the transducer unit 41, and simultaneously enables the drive shaft 42 to be rotated.

Ultrasonic scanning (scan) in the medical tube 1 is performed in such a way that the ultrasonic transducer 411 disposed in a housing 412 scans living tissues using transmitted and received ultrasonic waves in a substantially radial direction by transferring a rotational motion of the motor inside the drive unit 71 to the drive shaft 42 and rotating the housing 412 fixed to the distal end of the drive shaft 42. In addition, the medical tube 1 is entirely pulled to the proximal side, and the ultrasonic transducer 411 is moved in the longitudinal direction. In this manner, it is possible to scan any desired position so as to obtain a cross-sectional image of the surrounding tissue body at a viewing angle of 360 degrees in the axial direction inside the blood vessel.

Next, an operation performed when a lumen is internally observed using the medical tube 1 according to the present embodiment will be described.

First, before the sheath 2 of the medical tube 1 is inserted into the lumen, a priming operation for filling the inside of the medical tube 1 with the physiological saline is performed. Performing the priming operation removes air inside the medical tube 1, and prevents the air from entering the inside of the lumen such as the blood vessel.

In order to perform the priming operation, the hub 31 is brought into a state of being pulled furthest out from the unit connector 37 to a user's hand side, that is, a state where the inner tube 34 is pulled furthest out from the outer tube 32 (refer to Fig. 4). In this state, for example, the physiological saline is injected using a syringe via an instrument having a tube connected to the port 311 of the hub 31 and a three-way stopcock (not illustrated). The injected physiological saline fills the inside of the sheath 2 sequentially from the hub 31. If the inside of the medical tube 1 is completely filled with the physiological saline, the physiological saline is released from the priming port 231 formed in the filling liquid inlet and outlet member 23 (refer to Fig. 3) of the sheath 2. In this manner, filling of the physiological saline is confirmed. Performing the priming operation removes the air inside the medical tube 1, and prevents the air from entering the inside of the lumen.

Next, as illustrated in Fig. 2, the medical tube 1 is connected to the external drive device 7 covered with a sterilized polyethylene bag (not illustrated). That is, the joint 50 (refer to Fig. 5) of the hub 31 of the medical tube 1 is connected to the joint connection portion 712 of the drive unit 71. This enables a signal to be transmitted and received between the transducer unit 41 and the external drive device 7, and simultaneously enables the drive shaft 42 to be rotated. Then, if the unit connector 37 is fitted to the holding portion 73, the connection is completed.

Next, the drive unit 71 is moved to the distal side along the groove rail 76 on the base 75, the hub 31 being pressed into the distal side. In this manner, the inner tube 34 is brought into a state of being pressed furthest into the outer tube 32 (refer to Fig. 1). In this state, the sheath 2 is inserted into a body, and insertion of the distal end of the sheath 2 is stopped after crossing a target lesion.

Next, after the medical tube 1 is caused to reach a target site inside the lumen, a position of the sheath 2 is fixed. In this state, a pull-back operation is performed while the drive shaft 42 is rotated by the drive unit 71. In this manner, it is possible to acquire an image in the axial direction of the lumen.

The pull-back operation can be performed in such a way that the control unit 79 operates the moving means 72 connected to the rear end portion of the medical tube 1. Acquired data is displayed as image data on the display unit 78 after the data is digitally processed by the control unit 79.

Then, after the pull-back operation is performed, the hub 31 is pressed into the distal side again, and the imaging core 4 is moved forward. Thereafter, the medical tube 1 is pulled out from the inside of the lumen, and the operation of the medical tube 1 is completed.

Next, a processing apparatus 100 for thermally processing the outer tube 32 or the inner tube 34 will be described. Here, as an example, a case where the outer tube distal portion 321 of the outer tube 32 is thermally processed will be described.

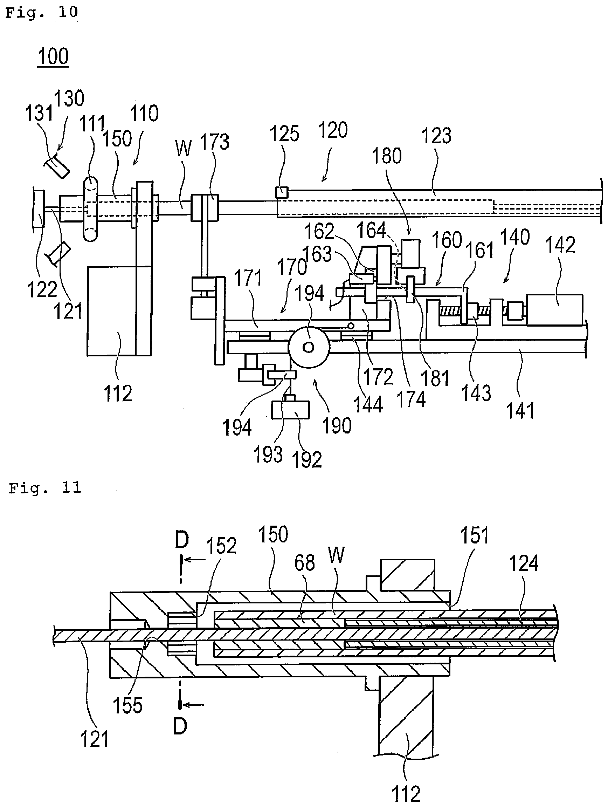

As illustrated in Fig. 10, the processing apparatus 100 includes a workpiece processing unit 110 which thermally processes an extrusion-molded tubular workpiece W, a workpiece holding unit 120 for holding the workpiece W, a cooling unit 130 for cooling the heated workpiece W, and an operation mechanism 140 for moving the workpiece W.

The workpiece processing unit 110 includes a mold 150, a high frequency coil 111 for heating the mold 150, and a mold holding stand 112 for holding the mold 150.

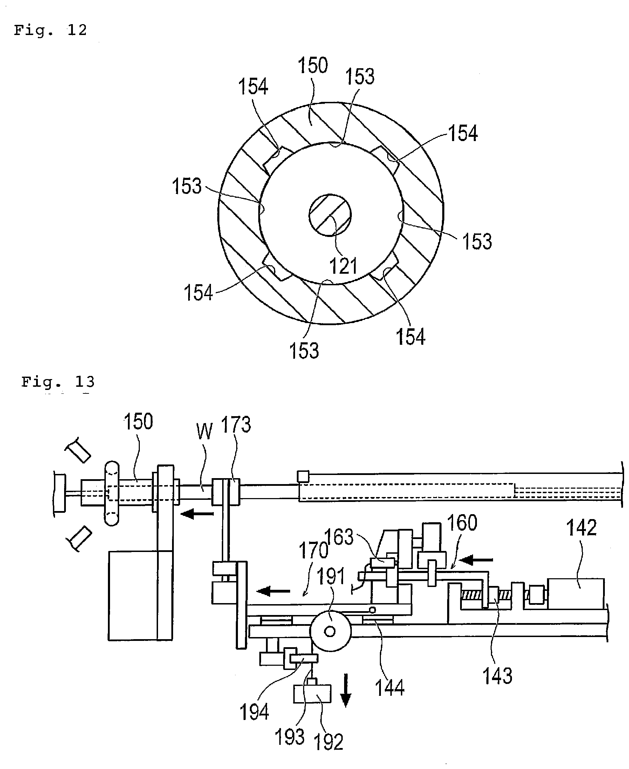

As illustrated in Figs. 11 and 12, the mold 150 has a substantially cylindrical shape. An insertion hole 151 into which the workpiece W is inserted is formed on one side thereof, and a cavity 152 for thermally processing the workpiece W is formed to the rear of the insertion hole 151. The cavity 152 is formed to have an inner diameter smaller than that of the insertion hole 151. Four convex portions 153 arrayed side by side at equal intervals in the circumferential direction are formed on an inner peripheral surface thereof, and a concave portion 154 is formed between the four convex portions 153. The convex portion 153 is a portion for molding the groove portion 321B of the outer tube distal portion 321, and the concave portion 154 is a portion for molding the projection portion 321A of the outer tube distal portion 321. In addition, a through-hole 155 which is coaxial with the inner diameter of the cavity 152 penetrates the mold 150 from a side opposite to the insertion hole 151. The mold 150 is formed of a magnetic metal material so that the mold 150 can be heated by the high frequency coil 111.

As illustrated in Fig. 10, the high frequency coil 111 is arranged so as to surround the outside of the portion where the cavity 152 of the mold 150 is formed, and is connected to a high frequency induction heating and oscillating device (not illustrated) which can supply a high frequency current to the high frequency coil 111. If the high frequency current flows in the high frequency coil 111, an induced electromotive force generated in the high frequency coil 111 generates eddy current in the adjacent mold 150 formed of the magnetic metal material. In this manner, it is possible to generate Joule heat. Applying high frequency induction heating enables the mold 150 to be heated in a non-contact manner in a short time. Accordingly, a tact time can be shortened, and the control is facilitated, thereby improving productivity.

The cooling unit 130 includes an air blowing nozzle 131 which can blow cooling air particularly to a portion close to the high frequency coil 111, within the outer periphery of the mold 150.

As illustrated in Figs. 10 and 11, the workpiece holding unit 120 includes a core metal 121 which penetrates the inside of the workpiece W and holds the workpiece W, a V-groove guide 123 which holds the workpiece W substantially horizontally so as to be movable in the axial direction, a spacer tube pressing tube 124 which presses the spacer tube 68 from the proximal side, and a workpiece detection sensor 125 which is fixed to the V-groove guide 123 and can detect the workpiece W.

The core metal 121 extends from a core metal stopper 122 fixedly arranged outside the mold 150 through the through-hole 155 of the mold 150, and penetrates the inside of the spacer tube 68 arranged inside the workpiece W. The core metal 121 serves to hold an axis of the workpiece W at a proper position when the workpiece W is thermally processed, and to hold the workpiece W so as not to be deformed in such a way that the workpiece W receives a force from the radially outside and escapes to the radially inside. The core metal stopper 122 serves to hold the core metal 121, and to prevent the core metal 121 from being simultaneously removed from the mold 150 when the workpiece W is removed from the mold 150 after the thermal processing.

The spacer tube pressing tube 124 is arranged inside the workpiece W, holds the spacer tube 68 so as to be pressed toward the distal side, and prevents the spacer tube 68 from escaping to the proximal side when the workpiece W is thermally processed.

The operation mechanism 140 includes a base 141, a motor 142 arranged on the base 141, a ball screw 143 which converts a rotational motion of the motor 142 into a linear motion, a first movement portion 160 which can be linearly moved by the ball screw 143, a second movement portion 170 which is disposed on the base 141 so to be movable via an LM guide 144, a connection portion 180 which connects the first movement portion 160 and the second movement portion 170, and a pressing force generation portion 190 which applies a constant force to the second movement portion 170.

The first movement portion 160 includes an extension portion 161 which is connected to the ball screw 143 and extends toward the second movement portion 170, a collar 162 which is fixed to the extension portion 161, and a detection switch 163 which can come into contact with the second movement portion 170 and detects a distance from the second movement portion 170.

The second movement portion 170 includes a second movement portion main body 171 on which the LM guide 144 is placed, a rising portion 172 which rises from the second movement portion main body 171, and a first air chuck 173 which is fixed to the second movement portion main body 171 and can grip the workpiece W using compressed air.

A passage 174 through which the extension portion 161 of the first movement portion 160 can pass and the collar 162 cannot pass is formed in the rising portion 172. The collar 162 and the detection switch 163 of the first movement portion 160 are arranged on the further forward side (side toward the mold) from the passage 174.

The connection portion 180 includes a second air chuck 181 which is fixed to the rising portion 172 of the second movement portion 170 and can grip the extension portion 161 of the first movement portion 160 using the compressed air. A positioning groove 164 to which the second air chuck 181 can be fitted is formed in a portion of the extension portion 161 which is gripped by the second air chuck 181.

The pressing force generation portion 190 includes a pulley 191 which is rotatably fixed to the base 141, a weight 192, a wire 193 which connects the second movement portion main body 171 and the weight 192 via the pulley 191, and a third air chuck 194 which is connected to the base 141 and can grip the wire 193 using the compressed air.

Next, a method for thermally processing the workpiece W using the above-described processing apparatus 100 will be described.

First, the spacer tube 68 and the spacer tube pressing tube 124 are installed inside the workpiece W to be arranged in the V-groove guide 123. The core metal 121 extending from the mold 150 is caused to penetrate the inside of the spacer tube 68. At this time, the first air chuck 173 is in an opened state without gripping the workpiece W.

Then, the first movement portion 160 and the second movement portion 170 are connected to each other by fitting the second air chuck 181 of the connection portion 180 to the positioning groove 164, in a state where the first movement portion 160 and the second movement portion 170 are moved rearward in a direction away from the mold 150. At this time, the collar 162 and the detection switch 163 of the first movement portion 160 are in contact with the rising portion 172 of the second movement portion 170. In addition, the third air chuck 194 of the pressing force generation portion 190 is closed so as to grip the wire 193, thereby preventing descending of the weight 192.

In this state, if an operator manually inserts the workpiece W into the insertion hole 151 of the mold 150 and pushes a start button (not illustrated) disposed in the processing apparatus 100, the current flows in the high frequency coil 111 to heat the mold 150, and the first air chuck 173 is closed so as to grip the workpiece W.

Then, as illustrated in Fig. 13, the third air chuck 194 is opened so that the wire 193 is movable. The motor 142 is driven and rotated, and the ball screw 143 causes the first movement portion 160 to move forward in a direction toward the mold. At this time, the first movement portion 160 and the second movement portion 170 are connected to each other. Accordingly, the second movement portion 170 also moves forward together along the LM guide 144, and the weight 192 moves downward in response to the movement of the second movement portion 170. The first air chuck 173 is fixed to the second movement portion 170. Accordingly, if the second movement portion 170 is moved, the workpiece W gripped by the first air chuck 173 is also moved so as to be pressed toward the mold 150. Meanwhile, the detection switch 163 fixed to the first movement portion 160 is in a switched-on state due to being in contact with the second movement portion 170.

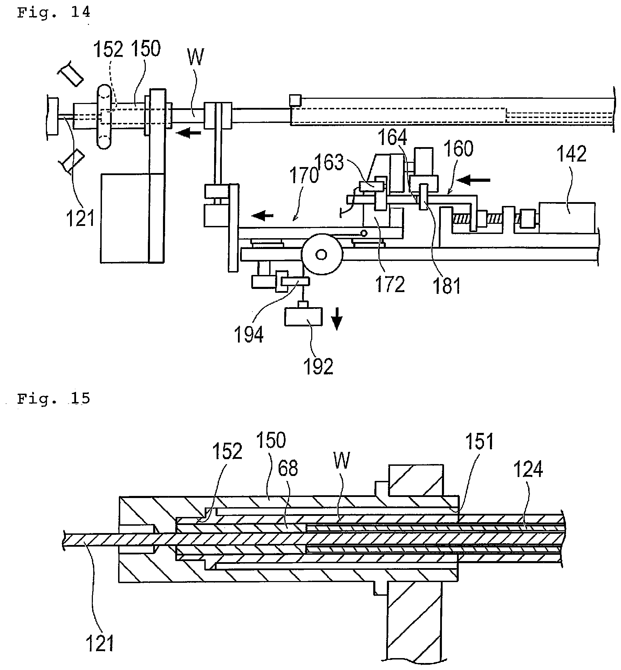

After the first movement portion 160 is moved by a predetermined length or for a predetermined period of time, the second air chuck 181 is opened, and the second movement portion 170 is separated from the first movement portion 160, as illustrated in Fig. 14. The motor 142 is rotated even after the separation. Therefore, the first movement portion 160 continues to move in a forward movement direction. In contrast, the second movement portion 170 no longer receives the force from the first movement portion 160, but a load of the weight 192 causes the second movement portion 170 to move forward in the direction toward the mold. The weight 192 causes the distal portion of the workpiece W to be pressed into the cavity 152 having a smaller inner diameter in the rear of the mold 150 by using a desirable constant load. The movement speed becomes slower than that of the first movement portion 160 due to a reaction force thereof, and the detection switch 163 is separated from the rising portion 172, thereby the switch being turned off. The workpiece W is heated inside the mold 150 so that a portion in contact with the mold 150 melts or is softened. As illustrated in Fig. 15, a constant load applied by the weight 192 causes the workpiece W to be deformed and pressed along an inner surface shape of the cavity 152. At this time, the spacer tube 68 is fixedly and integrally attached to the workpiece W in such a way that a portion of the spacer tube 68 melts or is softened. Then, the core metal 121 passes through the inside of the workpiece W and the spacer tube 68. Accordingly, the workpiece W and the spacer tube 68 are pressed into the mold 150 while the proper axes thereof are maintained, thereby maintaining high processing accuracy.

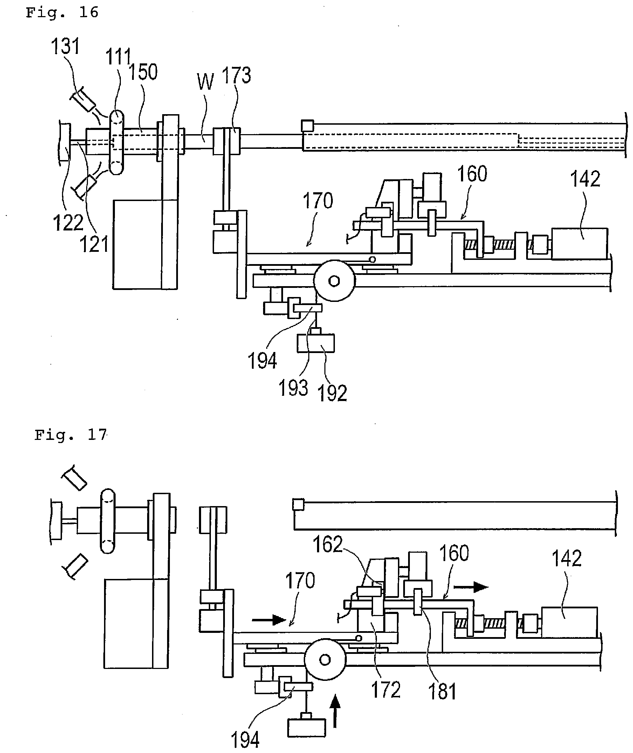

If a predetermined time (few seconds) elapses after the detection switch 163 is turned off, the third air chuck 194 is closed. If the third air chuck 194 is closed, the wire 193 is gripped, descending of the weight 192 is stopped, and the movement of the second movement portion 170 is stopped, as illustrated in Fig. 16. Thereafter, the rotation of the motor 142 is stopped, and the movement of the first movement portion 160 is also stopped. Then, supply of the current to the high frequency coil 111 is stopped to stop the heating. The air blowing nozzle 131 is driven to blow the air to the mold 150, and the mold 150 is cooled.

Thereafter, when the predetermined time has elapsed, the air blowing nozzle 131 is stopped, and the first air chuck 173 is opened. This enables an operator to manually pull the workpiece W out from the mold 150. When the workpiece W is pulled out, the core metal 121 is fixed by the core metal stopper 122. Accordingly, it is possible to pull out the thermally processed workpiece W while the core metal 121 is left behind.

When the first movement portion 160 and the second movement portion 170 are brought into a processing-available state again, the motor 142 is reversely rotated in a state where the second air chuck 181 and the third air chuck 194 are opened, as illustrated in Fig. 17, and the first movement portion 160 is moved rearward. If the first movement portion 160 is moved rearward and the collar 162 of the first movement portion 160 comes into contact with the rising portion 172 of the second movement portion 170, and the second movement portion 170 is also moved rearward by receiving the force from the collar 162. In this manner, the second movement portion 170 can be moved to its original state.

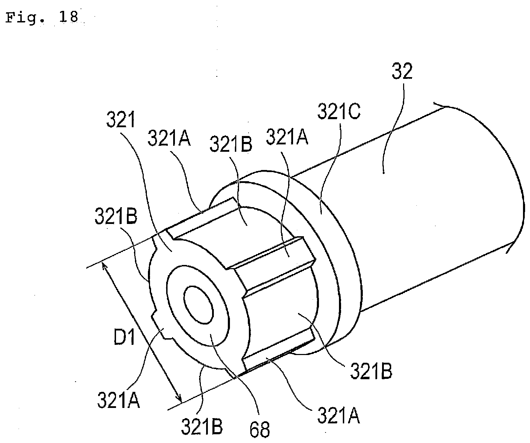

As illustrated in Fig. 18, the concave portion 154 and the convex portion 153 (refer to Fig. 12) inside the cavity 152 of the mold 150 are transcribed into the outer tube distal portion 321 where the workpiece W is thermally processed and molded, thereby forming the projection portions 321A and the groove portions 321B to be arrayed side by side in the circumferential direction. Then, a material melted during the thermal processing using the mold 150 flows into the insertion hole 151 from the cavity 152, thereby forming the annular portion 321C on the proximal side of the projection portion 321A. In this manner, the outer tube 32 including the outer tube distal portion 321 having high dimensional accuracy in the outer diameter D1 can be formed.

The outer tube proximal portion 322 and the inner tube proximal portion 341 are also similarly thermally processed. However, the mold 150, the core metal 121, and the weight 192 may be appropriately replaced depending on portions to be processed. In addition, processing conditions such as a processing time period may also be appropriately changed. Then, the spacer tube 68 is not disposed inside the outer tube proximal portion 322 and the inner tube proximal portion 341. Accordingly, the core metal 121 is used so as to directly come into contact with the outer tube 32 or the inner tube 34.

Then, as illustrated in Fig. 8, the thermally processed outer tube distal portion 321 is fitted to the inner peripheral surface 651 of the outer tube holding portion 65. As illustrated in Fig. 9, the thermally processed outer tube proximal portion 322 is fitted to the inner peripheral surface 611 of the unit connector main body 61. As illustrated in Fig. 6, the thermally processed inner tube proximal portion 341 is fitted to the inner peripheral surface 551 of the hub main body 55. The drive shaft 42 is rotatably arranged to penetrate the inside of the outer tube 32, the inner tube 34, the hub main body 55, the unit connector main body 61, and the outer tube holding portion 65.

As described above, the medical tube 1 according to the present embodiment is configured so that the outer peripheral surface of the end portion of the tube body (outer tube 32 or inner tube 34) is thermally processed into a different shape after extrusion molding. Therefore, dimensional accuracy in the outer diameter of the outer peripheral surface is improved, thereby enabling the outer peripheral surface to be fitted to an injection-molded containing body having high dimensional accuracy (hub main body 55, unit connector main body 61, or outer tube holding portion 65) by using a more desirable clearance. Therefore, axes of the extrusion-molded component and the injection-molded component are less likely to deviate from each other, relative positions of the two components are less likely to be tilted, or lengths for fitting are less likely to be changed, thereby improving performance of the medical tube 1. In addition, the above-described relative positional deviation decreases, thereby facilitating automation of an assembly process and improving productivity.

In addition, at least one (four in the present embodiment) of the groove portions 321B, 322B, and 341B is formed on the outer peripheral surface of the end portion of the tube body (outer tube 32 or inner tube 34) along the axial direction of the tube body. Accordingly, when the outer surface is fitted to the containing body (hub main body 55, unit connector main body 61, or outer tube holding portion 65), the projection portions 321A, 322A, and 341A formed between the groove portions 321B, 322B, and 341B can be respectively deformed. In this manner, it is easy to decrease the positional deviation by causing the axes of the tube body and the containing body to coincide with each other. In addition, when the tube body is fitted to the containing portion, the groove portions 321B, 322B, and 341B decrease frictional resistance, thereby facilitating the fitting. In addition, since the groove portions 321B, 322B, and 341B are provided, it is easy to spread the adhesives 321D, 322D, and 341D throughout the fitting portion, when the adhesives 321D, 322D, and 341D are used. Moreover, excess adhesives 321D, 322D, and 341D can be released to the groove portions 321B, 322B, and 341B, thereby enabling favorable adhesion.

In addition, the outer peripheral surface of the end portion of the tube body (outer tube 32 or inner tube 34) has a rotational symmetric shape in a cross section orthogonal to the axial direction of the tube body. Accordingly, when the tube body is fitted to the containing body (hub main body 55, unit connector main body 61, or outer tube holding portion 65), the projection portions 321A, 322A, and 341A formed between the groove portions 321B, 322B, and 341B are respectively uniformly deformed. In this manner, it is easy to decrease the positional deviation by causing the axes of the tube body and the containing body to coincide with each other.

In addition, the medical tube 1 has the drive shaft 42 in which the ultrasonic transducer 411 acquiring image information is fixed to the end portion, and which transfers the mechanical drive force to the ultrasonic transducer 411. However, the positional deviation between the tube body (outer tube 32 or inner tube 34) and the containing body (hub main body 55, unit connector main body 61, or outer tube holding portion 65) is adapted to be smaller. Accordingly, when the drive shaft 42 is rotated, sliding resistance is less likely to occur locally between the drive shaft 42 and other members (tube body, containing body, or sheath 2). Therefore, it is possible to prevent uneven rotation. If uneven rotation occurs, when an image is displayed during diagnosis with a catheter, characteristics which are anatomically different from those of the original lumen are displayed. However, since uneven rotation can be prevented from occurring, characteristics which are anatomically close to those of the original lumen can be displayed.

In addition, both end portions of the outer tube 32 arranged on the proximal side of the sheath 2 and the proximal portion of the inner tube 34 which is arranged on the distal side of the hub 31 and is relatively movable with respect to the outer tube 32 in response to the movement of the hub 31 are thermally processed so as to improve dimensional accuracy. Accordingly, even when the outer tube 32 and the inner tube 34 are relatively moved, proper axes or positions can be maintained, thereby improving performance of the medical tube 1 for imaging diagnosis.

The present invention is not limited to only the above-described embodiment, and can be modified in various ways by those skilled in the art within the technical scope of the present invention. For example, in the above-described embodiment, a case has been described where the present invention is applied to the ultrasonic catheter. However, the present invention can be applied to an optical probe for a diagnostic apparatus such as an optical coherence tomography diagnostic apparatus and an optical frequency domain imaging diagnosis apparatus, or an endoscopic system. As long as a tubular body is provided, the present invention can be applied to various medical tubes.

In addition, the present embodiment adopts a configuration in which the tube body (inner tube 34) on the proximal side enters the inside of the tube body (outer tube 32) on the distal side. However, a configuration may be adopted in which the tube body on the distal side enters the inside of the tube body on the proximal side.

### Reference Signs List

1 medical tube
2 sheath
31 hub
32 outer tube (first tube body)
34 inner tube (second tube body)
42 drive shaft
55 hub main body (containing portion)
61 unit connector main body (containing portion)
65 outer tube holding portion (containing portion)
321 outer tube distal portion
321A, 322A, 341A projection portion
321B, 322B, 341B groove portion
322 outer tube proximal portion
341 inner tube proximal portion
411 ultrasonic transducer (signal transmitting and receiving unit)
551, 611, 651 inner peripheral surface

## Claims

1. A medical tube (1) comprising:
an extrusion-molded tube body (32, 34) comprising an outer peripheral surface of an end portion (321, 322, 341) of the tube body (32, 34);
an injection-molded containing body (55, 61, 65) including an inner peripheral surface (551, 611, 651) to which the outer peripheral surface of the end portion (321, 322, 341) of the tube body (32, 34) is fitted,
**characterized in that** the outer peripheral surface of the end portion (321, 322, 341) of the tube body (32, 34) has at least one groove portion (321B, 322B, 341B) and at least one protruding portion (321A, 322A, 341A) extending along a longitudinal axial direction of the tube body (32, 34), wherein the at least one protruding portion (321A, 322A, 341A) has a predetermined outer diameter (D1, D2, D3), and wherein the at least one groove portion (321B, 322B, 341B) and the at least one protruding portion (321A, 322A, 341A) of the outer peripheral surface of the end portion (321, 322, 341) of the tube body (32, 34) are formed by means of thermal processing using a mold after extrusion molding.

2. The medical tube (1) according to Claim 1,
wherein the outer peripheral surface of the end portion (321, 322, 341) of the tube body (32, 34) with the predetermined outer diameter (Dl, D2, D3) has a rotational symmetric shape in a cross section orthogonal to the axial direction of the tube body (32, 34).

3. The medical tube (1) according to anyone of Claims 1 or 2, further comprising:
a drive shaft (42) that rotatably penetrates the inside of the tube body (32, 34) and the containing body (55, 61, 65), to an end portion of which a signal transmitting and receiving portion (411) for acquiring image information is fixed, and that transfers a mechanical drive force to the signal transmitting and receiving portion (411).

4. The medical tube (1) according to Claim 3, further comprising:
a sheath (2) in which the signal transmitting and receiving portion (411) and the drive shaft (42) are arranged to be movable thereinside, and which is inserted into a lumen;
a hub (31) which moves while holding the drive shaft (42) so as to move the drive shaft (42) in the axial direction of the sheath (2);
a first tube body (32) which is arranged on a proximal side of the sheath (2); and
a second tube body (34) which is arranged on a distal side of the hub (31), and which can relatively move an inner portion or an outer portion of the first tube body (32) with respect to the first tube body (32) in accordance with a movement of the hub (31).

5. A method for producing a medical tube (1) that has an extrusion-molded tube body (32, 34) comprising an outer peripheral surface of an end portion (321, 322, 341) of the tube body (32, 34) and an injection-molded containing body (55, 61, 65) including an inner peripheral surface (551, 611, 651) to which the outer peripheral surface of the end portion (321, 322, 341) of the tube body (32, 34) is fitted, the method comprising:
a process of producing the tube body (32, 34) by molding an outer peripheral surface of an end portion of an extrusion-molded and tubular workpiece into a different shape by means of thermal processing; and
a process of fitting the end portion (321, 322, 341) of the thermally processed tube body (32, 34) to the containing body (55, 61, 65),
**characterized in that** in the process of producing the tube body (32, 34), the outer peripheral surface of the end portion (321, 322, 341) of the tube body (32, 34) has at least one groove portion (321B, 322B, 341B) and at least one protruding portion (321A, 322A, 341A) extending along a longitudinal axial direction of the tube body (32, 34), wherein the at least one protruding portion (321A, 322A, 341A) has a predetermined outer diameter (D1, D2, D3), and wherein the at least one groove portion (321B, 322B, 341B) and the at least one protruding portion (321A, 322A, 341A) of the outer peripheral surface of the end portion (321, 322, 341) of the tube body (32, 34) are formed by means of thermal processing using a mold after extrusion molding.

6. The method for producing a medical tube (1) according to Claim 5,
wherein in the process of producing the tube body (32, 34), the outer peripheral surface of the end portion (321, 322, 341) of the tube body (32, 34) with the predetermined outer diameter (D1, D2, D3) is formed into a rotational symmetric shape in a cross section orthogonal to the axial direction of the tube body (32, 34).

7. The method for producing a medical tube (1) according to any one of Claims 5 to 6, further comprising:
a process of arranging a drive shaft (42) that rotatably penetrates the inside of the tube body (32, 34) and the containing body (55, 61, 65), to an end portion of which a signal transmitting and receiving portion (411) for acquiring image information is fixed, and that transfers a mechanical drive force to the signal transmitting and receiving portion (411), so as to rotatably penetrate the inside of the tube body (32, 34) and the containing body (55, 61, 65).

8. The method for producing a medical tube (1) according to Claim 7,
wherein the medical tube (1) includes:
a sheath (2) in which the signal transmitting and receiving portion (411) and the drive shaft (42) are arranged to be movable thereinside, and which is inserted into a lumen;
a hub (31) which moves while holding the drive shaft (42) so as to move the drive shaft (42) in the axial direction of the sheath (2);
a first tube body (32) which is arranged on a proximal side of the sheath (2); and
a second tube body (34) which is arranged on a distal side of the hub (31), and which can relatively move an inner portion or an outer portion of the first tube body (32) with respect to the first tube body (32) in accordance with a movement of the hub (31).

## Patentansprüche

1. Medizinischer Schlauch (1), umfassend:
einen stranggepressten Schlauchkörper (32, 34), der eine äußere Umfangsfläche eines Endabschnitts (321, 322, 341) des Schlauchkörpers (32, 34) umfasst;
einen spritzgegossenen Aufnahmekörper (55, 61, 65), der eine innere Umfangsfläche (551, 611, 651) umfasst, an welche die äußere Umfangsfläche des Endabschnitts (321, 322, 341) des Schlauchkörpers (32, 34) angepasst ist,
**dadurch gekennzeichnet, dass** die äußere Umfangsfläche des Endabschnitts (321, 322, 341) des Schlauchkörpers (32, 34) mindestens einen Rillenabschnitt (321B, 322B, 341B) und mindestens einen vorstehenden Abschnitt (321A, 322A, 341A) aufweist, der sich entlang einer axialen Längsrichtung des Schlauchkörpers (32, 34) erstreckt, wobei der mindestens eine vorstehende Abschnitt (321A, 322A, 341A) einen vorbestimmten Außendurchmesser (Dl, D2, D3) aufweist, und wobei der mindestens eine Rillenabschnitt (321B, 322B, 341B) und der mindestens eine vorstehende Abschnitt (321A, 322A, 341A) der äußeren Umfangsfläche des Endabschnitts (321, 322, 341) des Schlauchkörpers (32, 34) mittels thermischer Behandlung unter Verwendung einer Form nach dem Strangpressen gebildet werden.

2. Medizinischer Schlauch (1) nach Anspruch 1,
wobei die äußere Umfangsfläche des Endabschnitts (321, 322, 341) des Schlauchkörpers (32, 34) mit dem vorbestimmten Außendurchmesser (Dl, D2, D3) eine rotationssymmetrische Form in einem Querschnitt orthogonal zu der axialen Richtung des Schlauchkörpers (32, 34) aufweist.

3. Medizinischer Schlauch (1) nach einem der Ansprüche 1 oder 2, ferner umfassend:
eine Antriebswelle (42), welche drehbar in das Innere des Schlauchkörpers (32, 34) und des Aufnahmekörpers (55, 61, 65) eindringt, wobei an deren einem Endabschnitt ein Signalsende- und -empfangsabschnitt (411) zum Erfassen von Bildinformationen angebracht ist, und die eine mechanische Antriebskraft auf den Signalsende- und -empfangsabschnitt (411) überträgt.

4. Medizinischer Schlauch (1) nach Anspruch 3, ferner umfassend:
eine Hülle (2), in welcher der Signalsende- und - empfangsabschnitt (411) und die Antriebswelle (42) so angeordnet sind, dass sie darin beweglich sind, und die in ein Lumen eingeführt ist;
eine Nabe (31), die sich bewegt, während sie die Antriebswelle (42) hält, um so die Antriebswelle (42) in der axialen Richtung der Hülle (2) zu bewegen;
einen ersten Schlauchkörper (32), der auf einer proximalen Seite der Hülle (2) angeordnet ist; und
einen zweiten Schlauchkörper (34), der an einer distalen Seite der Nabe (31) angeordnet ist und der relativ einen inneren Abschnitt oder einen äußeren Abschnitt des ersten Schlauchkörpers (32) in Bezug auf den ersten Schlauchkörper (32) entsprechend einer Bewegung der Nabe (31) bewegen kann.

5. Verfahren zur Herstellung eines medizinischen Schlauchs (1), welcher einen stranggepressten Schlauchkörper (32, 34) aufweist, der eine äußere Umfangsfläche eines Endabschnitts (321, 322, 341) des Schlauchkörpers (32, 34) und einen spritzgegossenen Aufnahmekörper (55, 61, 65) mit einer inneren Umfangsfläche (551, 611, 651) umfasst, an welche die äußere Umfangsfläche des Endabschnitts (321, 322, 341) des Schlauchkörpers (32, 34) angepasst ist, wobei das Verfahren umfasst:
ein Verfahren zum Herstellen des Schlauchkörpers (32, 34) durch Formen einer äußeren Umfangsfläche eines Endabschnitts eines stranggepressten und schlauchförmigen Werkstücks in eine andere Form mittels thermischer Bearbeitung; und
ein Verfahren zum Anpassen des Endabschnitts (321, 322, 341) des thermisch bearbeiteten Schlauchkörpers (32, 34) an den Aufnahmekörper (55, 61, 65),
**dadurch gekennzeichnet, dass** bei dem Verfahren zur Herstellung des Schlauchkörpers (32, 34) die äußere Umfangsfläche des Endabschnitts (321, 322, 341) des Schlauchkörpers (32, 34) mindestens einen Rillenabschnitt (321B, 322B, 341B) und mindestens einen vorstehenden Abschnitt (321A, 322A, 341A) aufweist, der sich entlang einer axialen Längsrichtung des Schlauchkörpers (32, 34) erstreckt, wobei der mindestens eine vorstehende Abschnitt (321A, 322A, 341A) einen vorbestimmten Außendurchmesser (Dl, D2, D3) aufweist, und wobei der mindestens eine Rillenabschnitt (321B, 322B, 341B) und der mindestens eine vorstehende Abschnitt (321A, 322A, 341A) der äußeren Umfangsfläche des Endabschnitts (321, 322, 341) des Schlauchkörpers (32, 34) mittels thermischer Bearbeitung unter Verwendung einer Form nach dem Strangpressen gebildet werden.

6. Verfahren zur Herstellung eines medizinischen Schlauchs (1) nach Anspruch 5,
wobei in dem Verfahren zur Herstellung des Schlauchkörpers (32, 34) die äußere Umfangsfläche des Endabschnitts (321, 322, 341) des Schlauchkörpers (32, 34) mit dem vorbestimmten Außendurchmesser (Dl, D2, D3) in eine rotationssymmetrische Form in einem Querschnitt orthogonal zu der axialen Richtung des Schlauchkörpers (32, 34) geformt wird.

7. Verfahren zur Herstellung eines medizinischen Schlauches (1) nach einem der Ansprüche 5 bis 6, ferner umfassend:
ein Verfahren zum Anordnen einer Antriebswelle (42), welche drehbar in das Innere des Schlauchkörpers (32, 34) und des Aufnahmekörpers (55, 61, 65) eindringt, wobei an deren einem Endabschnitt ein Signalsende- und - empfangsabschnitt (411) zum Erfassen von Bildinformationen angebracht ist, und die eine mechanische Antriebskraft auf den Signalsende- und -empfangsabschnitt (411) überträgt, sodass sie drehbar in das Innere des Schlauchkörpers (32, 34) und des Aufnahmekörpers (55, 61, 65) eindringt.

8. Verfahren zur Herstellung eines medizinischen Schlauchs (1) nach Anspruch 7,
wobei der medizinische Schlauch (1) umfasst:
eine Hülle (2), in welcher der Signalsende- und - empfangsabschnitt (411) und die Antriebswelle (42) so angeordnet sind, dass sie darin beweglich sind, und die in ein Lumen eingeführt ist;
eine Nabe (31), die sich bewegt, während sie die Antriebswelle (42) hält, um so die Antriebswelle (42) in der axialen Richtung der Hülle (2) zu bewegen;
einen ersten Schlauchkörper (32), der auf einer proximalen Seite der Hülle (2) angeordnet ist; und
einen zweiten Schlauchkörper (34), der an einer distalen Seite der Nabe (31) angeordnet ist und der relativ einen inneren Abschnitt oder einen äußeren Abschnitt des ersten Schlauchkörpers (32) in Bezug auf den ersten Schlauchkörper (32) entsprechend einer Bewegung der Nabe (31) bewegen kann.

## Revendications

1. Tube médical (1) comprenant :
un corps de tube moulé par extrusion (32, 34) comprenant une surface périphérique extérieure d'une partie d'extrémité (321, 322, 341) du corps de tube (32, 34) ;
un corps contenant moulé par injection (55, 61, 65) comprenant une surface périphérique intérieure (551, 611, 651) sur laquelle la surface périphérique extérieure de la partie d'extrémité (321, 322, 341) du corps de tube (32, 34) est montée,
**caractérisé en ce que** la surface périphérique extérieure de la partie d'extrémité (321, 322, 341) du corps de tube (32, 34) comprend au moins une partie de rainure (321B, 322B, 341B) et au moins une partie saillante (321A, 322A, 341A) s'étendant le long d'une direction axiale longitudinale du corps de tube (32, 34), où la au moins une partie saillante (321A, 322A, 341A) a un diamètre extérieur prédéterminé (D1, D2, D3), et où la au moins une partie de rainure (321B, 322B, 341B) et la au moins une partie saillante (321A, 322A, 341A) de la surface périphérique extérieure de la partie d'extrémité (321, 322, 341) du corps de tube (32, 34) sont formées par traitement thermique à l'aide d'un moule après moulage par extrusion.

2. Tube médical (1) selon la revendication 1,
dans lequel la surface périphérique extérieure de la partie d'extrémité (321, 322, 341) du corps de tube (32, 34) ayant le diamètre extérieur prédéterminé (D1, D2, D3) a une forme symétrique en rotation dans une section transversale orthogonale à la direction axiale du corps de tube (32, 34).

3. Tube médical (1) selon l'une quelconque des revendications 1 ou 2, comprenant en outre :
un arbre d'entraînement (42) qui pénètre en rotation à l'intérieur du corps de tube (32, 34) et du corps contenant (55, 61, 65), sur une partie d'extrémité duquel une partie d'émission et de réception de signal (411) destinée à acquérir une information d'image est fixée, et qui transfère une force d'entraînement mécanique à la partie d'émission et de réception de signal (411).

4. Tube médical (1) selon la revendication 3, comprenant en outre :
une gaine (2) dans laquelle la partie d'émission et de réception de signal (411) et l'arbre d'entraînement (42) sont disposés de manière à pouvoir se déplacer à l'intérieur de celle-ci, et qui est insérée dans une lumière ;
un embout (31) qui se déplace tout en maintenant l'arbre d'entraînement (42) de manière à déplacer l'arbre d'entraînement (42) dans la direction axiale de la gaine (2) ;
un premier corps de tube (32) qui est disposé sur un côté proximal de la gaine (2) ; et
un second corps de tube (34) qui est disposé sur un côté distal de l'embout (31), et qui peut déplacer de manière relative une partie intérieure ou une partie extérieure du premier corps de tube (32) par rapport au premier corps de tube (32) en fonction d'un mouvement de l'embout (31).

5. Procédé de production d'un tube médical (1) qui comprend un corps de tube moulé par extrusion (32, 34) comprenant une surface périphérique extérieure d'une partie d'extrémité (321, 322, 341) du corps de tube (32, 34) et un corps contenant moulé par injection (55, 61, 65) comprenant une surface périphérique intérieure (551, 611, 651) sur laquelle la surface périphérique extérieure de la partie d'extrémité (321, 322, 341) du corps de tube (32, 34) est adaptée, le procédé comprenant les étapes consistant à :
fabriquer le corps de tube (32, 34) en moulant une surface périphérique extérieure d'une partie d'extrémité d'une pièce tubulaire et moulée par extrusion en une forme différente par traitement thermique ; et
monter la partie d'extrémité (321, 322, 341) du corps de tube traité thermiquement (32, 34) sur le corps contenant (55, 61, 65),
**caractérisé en ce que**, dans le procédé de fabrication du corps de tube (32, 34), la surface périphérique extérieure de la partie d'extrémité (321, 322, 341) du corps de tube (32, 34) comprend au moins une partie de rainure (321B, 322B, 341B) et au moins une partie saillante (321A, 322A, 341A) s'étendant le long d'une direction axiale longitudinale du corps de tube (32, 34), où la au moins une partie saillante (321A, 322A, 341A) a un diamètre extérieur prédéterminé (D1, D2, D3), et où la au moins une partie de rainure (321B, 322B, 341B) et la au moins une partie saillante (321A, 322A, 341A) de la surface périphérique extérieure de la partie d'extrémité (321, 322, 341) du corps de tube (32, 34) sont formées par traitement thermique à l'aide d'un moule après moulage par extrusion.

6. Procédé de production d'un tube médical (1) selon la revendication 5,
où, dans le procédé de fabrication du corps de tube (32, 34), la surface périphérique extérieure de la partie d'extrémité (321, 322, 341) du corps de tube (32, 34) ayant le diamètre extérieur prédéterminé (D1, D2, D3) est formée en une forme symétrique en rotation dans une section transversale orthogonale à la direction axiale du corps de tube (32, 34).

7. Procédé de production d'un tube médical (1) selon l'une quelconque des revendications 5 à 6, comprenant en outre :
une étape consistant à disposer un arbre d'entraînement (42) qui pénètre en rotation à l'intérieur du corps de tube (32, 34) et du corps contenant (55, 61, 65), sur une partie d'extrémité duquel une partie d'émission et de réception de signal (411) destinée à acquérir une information d'image est fixée, et qui transfère une force d'entraînement mécanique à la partie d'émission et de réception de signal (411), de manière à pénétrer en rotation à l'intérieur du corps de tube (32, 34) et du corps contenant (55, 61, 65).

8. Procédé de production d'un tube médical (1) selon la revendication 7,
où le tube médical (1) comprend :
une gaine (2) dans laquelle la partie d'émission et de réception de signal (411) et l'arbre d'entraînement (42) sont disposés de manière à pouvoir se déplacer à l'intérieur de celle-ci, et qui est insérée dans une lumière ;
un embout (31) qui se déplace tout en maintenant l'arbre d'entraînement (42) de manière à déplacer l'arbre d'entraînement (42) dans la direction axiale de la gaine (2) ;
un premier corps de tube (32) qui est disposé sur un côté proximal de la gaine (2) ; et
un second corps de tube (34) qui est disposé sur un côté distal de l'embout (31), et qui peut déplacer de manière relative une partie intérieure ou une partie extérieure du premier corps de tube (32) par rapport au premier corps de tube (32) en fonction d'un mouvement de l'embout (31).
